# EUROPÄISCHE PATENTSCHRIFT

(11) **EP 2 495 487 B1**
(45) Veröffentlichungstag und Bekanntmachung des Hinweises auf die Patenterteilung: **11.06.2014**
(21) Anmeldenummer: 11156645.1
(22) Anmeldetag: 02.03.2011
(51) Int. Cl.: F21S 8/00, F21V 14/02, F21V 23/04, F21W 131/205, A61B 19/00, F21V 5/04, F21V 21/30, F21V 21/40, F21Y 101/02, F21Y 113/00

(54) **Operationsleuchte und Verfahren zum Ausleuchten einer Operationsstelle**
Operation lamp and method for illuminating an operation point
Lumière d'opération et procédé d'illumination d'un point d'opération

(43) Veröffentlichungstag der Anmeldung: 05.09.2012
(73) Patentinhaber: TRUMPF Medizin Systeme GmbH + Co. KG, 07318 Saalfeld (DE)
(72) Erfinder: Marka, Rudolf, 85737 Ismaning (DE); Rosenheimer, Rouven, 81377 München (DE)
(74) Vertreter: Prüfer & Partner GbR European Patent Attorneys

(56) Entgegenhaltungen:
- EP-A1- 2 136 128
- EP-A1- 2 215 987
- EP-A1- 2 283 790
- EP-A2- 0 299 196
- EP-A2- 0 422 331
- US-A- 5 068 767

## Beschreibung

Die Erfindung bezieht sich auf eine Operationsleuchte, insbesondere auf eine Operationsleuchte, die ein Leuchtfeld bereitstellt, dessen Leuchtfelddurchmesser sich nach einer Veränderung des Arbeitsabstands nicht verändert.

Um Operationen unter für den Operateur günstigen Bedingungen durchzuführen, ist unter anderem eine gute Ausleuchtung des Operationsfelds erforderlich. Dies kann üblicherweise durch Einstellen verschiedener Parameter der Operationsleuchte erreicht werden. Dazu sind in der Regel eine Position und eine Orientierung des Leuchtenkörpers, eine Fokussierung der Lichtstrahlen auf die Operationsstelle und eine Lichtstärke des abgestrahlten Lichts, also die Beleuchtungsstärke auf der Operationsstelle, einstellbar. Ein Verändern der Position und der Orientierung sowie die Leuchtfeldzusammenführung werden üblicherweise durch den Operateur selbst durchgeführt, der dazu den Leuchtenkörper an einem sterilen Handgriff nimmt, und an die gewünschte Position und in die gewünschte Orientierung schwenkt. Durch Verdrehen des sterilen Handgriffs wird dann üblicherweise die Leuchtfeldzusammenführung, d.h. der Abstand des Schnittpunkts der abgestrahlten Lichtstrahlen von dem Leuchtenkörper verstellt.

Die europäische Patentanmeldung EP 2 136128 A1 zeigt eine Leuchte, bei der die Leuchtfeldzusammenführung durch ein Ansteuern von verschiedenen Lichtquellen erfolgt, deren Lichtstrahlen auf verschiedene Schnittpunkte der Lichtstrahlen mit der Achse des Leuchtenkörpers gerichtet sind. Der Abstand zwischen dem Leuchtenkörper und den einzelnen Schnittpunkten ist dabei unterschiedlich. Die Leuchtfeldzusammenführung erfolgt also hierbei nicht mechanisch sondern durch bloße Ansteuerung der verschiedenen Lichtquellen.

Es gibt inzwischen Operationsleuchten, bei denen der Abstand zwischen dem Leuchtenkörper und der Operationsstelle gemessen wird, und die Lichtstärke des abgestrahlten Lichts entsprechend einer Arbeitsabstandsänderung korrigiert wird, so dass die zentrale Beleuchtungsstärke auf der Operationsstelle unverändert bleibt. Dabei wird aber nur die Beleuchtungsstärke, aber nicht der Leuchtfelddurchmesser angepasst, da die Lichtquellen in diesem System starr angeordnet sind, und Parallelstrahler, die dieses Problem teilweise lösen würden, mit diesem Optikkonzept nicht realisierbar sind.

Daraus ergibt sich die Aufgabe, eine Operationsleuchte bereitzustellen, die bei einer Veränderung des Arbeitsabstands den Leuchtfelddurchmesser unverändert beibehält.

Die Aufgabe wird durch einen Gegenstand mit den Merkmalen des Anspruchs 1 und ein Verfahren mit den Merkmalen des Anspruchs 14 gelöst. Weiterentwicklungen der Erfindung sind Gegenstand der abhängigen Ansprüche.

Eine Operationsleuchte zum Ausleuchten einer in einem gewählten Abstand angeordneten Operationsstelle ist dazu so ausgebildet, dass sie gemäß einem Aspekt zumindest einen Leuchtenkörper aufweist, der mindestens eine erste Lichtquelle und eine zweite Lichtquelle aufweist, wobei die Lichtquellen angepasst sind, jeweils ein erstes Leuchtfeld und ein zweites Leuchtfeld mit unterschiedlichen Durchmessern zu bilden, die ein resultierendes im Wesentlichen kreisförmiges Leuchtfeld mit einer Operationsleuchtennorm-gerechten Lichtverteilung mit einer voreingestellten normgerechten relativen Beleuchtungsstärke bei einem vorbestimmten Durchmesser ergeben, und eine Steuerungsvorrichtung für die Lichtquellen aufweist, die dazu angepasst ist, die Lichtstärke der ersten Lichtquelle und die Lichtstärke der zweiten Lichtquelle individuell anzusteuern, so dass bei dem gewählten Abstand die voreingestellte relative Beleuchtungsstärke bei dem vorbestimmten Durchmesser vorliegt. Die einzelnen Lichtstärken der Lichtquellen werden individuell so gesteuert, dass der Durchmesser, bei dem eine voreingestellte relative Beleuchtungsstärke des resultierenden Leuchtfelds vorliegt, bei einer nachträglichen Veränderung des vorher gewählten Abstands zwischen dem Leuchtenkörper und der Operationsstelle im Wesentlichen unverändert bleibt.

Die Erfindung wird nun unter Bezugnahme auf die beigefügten Zeichnungen anhand eines Ausführungsbeispiels näher erläutert.

Im Einzelnen zeigen:
- Fig. 1: eine perspektivische Darstellung einer Operationsleuchte;
- Fig. 2: eine Ansicht eines Leuchtenkörpers der Operationsleuchte von Fig. 1 von schräg unten;
- Fig. 3a: ein Diagramm einer Lichtverteilung einer Lichtquelle, die ein Leuchtfeld mit einem kleinen Leuchtfelddurchmesser bildet;
- Fig. 3b: ein Diagramm einer Lichtverteilung einer Lichtquelle, die ein Leuchtfeld mit einem großen Leuchtfelddurchmesser bildet;
- Fig. 3c: ein Diagramm einer Lichtverteilung, bei der sich die Leuchtfelder aus Fig. 3a und Fig. 3b überlagern;
- Fig. 3d: ein Diagramm einer Lichtverteilung, bei der sich die Leuchtfelder aus Fig. 3a und Fig. 3b überlagern und bei der die Lichtquellen verschiedene zentrale Beleuchtungsstärken aufweisen;
- Fig. 3e: ein Diagramm mit den überlagerten Lichtverteilungen aus den Fig. 3c und 3d;
- Fig. 4a: eine Schnittdarstellung des Leuchtenkörpers von Fig. 2 entlang der Schnittlinie A-A in Fig. 1 mit einer prin- zipiellen Darstellung eines Leuchtfelds einer ersten Lichtquelle;
- Fig. 4b: eine weitere Schnittdarstellung des Leuchtenkörpers von Fig. 2 entlang der Schnittlinie A-A in Fig. 1 mit einer prinzipiellen Darstellung eines Leuchtfelds einer zweiten Lichtquelle;
- Fig. 4c: eine weitere Schnittdarstellung des Leuchtenkörpers von Fig. 2 entlang der Schnittlinie A-A in Fig. 1 mit einer prinzipiellen Darstellung eines resultierenden Leuchtfelds der ersten und zweiten Lichtquelle;
- Fig. 4d: eine weitere Schnittdarstellung des Leuchtenkörpers von Fig. 2 entlang der Schnittlinie A-A in Fig. 1 mit einer prinzipiellen Darstellung eines resultierenden Leuchtfelds der ersten, zweiten, dritten und vierten Lichtquelle im Abstand l₁;
- Fig. 4e: eine weitere Schnittdarstellung des Leuchtenkörpers von Fig. 2 entlang der Schnittlinie A-A in Fig. 1 mit einer prinzipiellen Darstellung des resultierenden Leuchtfeld der ersten, zweiten, dritten und vierten Lichtquelle im Abstand l₂; und
- Fig. 5: eine weitere Schnittdarstellung des Leuchtenkörpers von Fig. 2 entlang der Schnittlinie A-A in Fig. 1 mit einer prinzipiellen Darstellung von Leuchtfeldern von zwei ersten Lichtquellen;
- Fig. 6: einen Handgriff der Operationsleuchte auf einer Handgriffaufnahme.

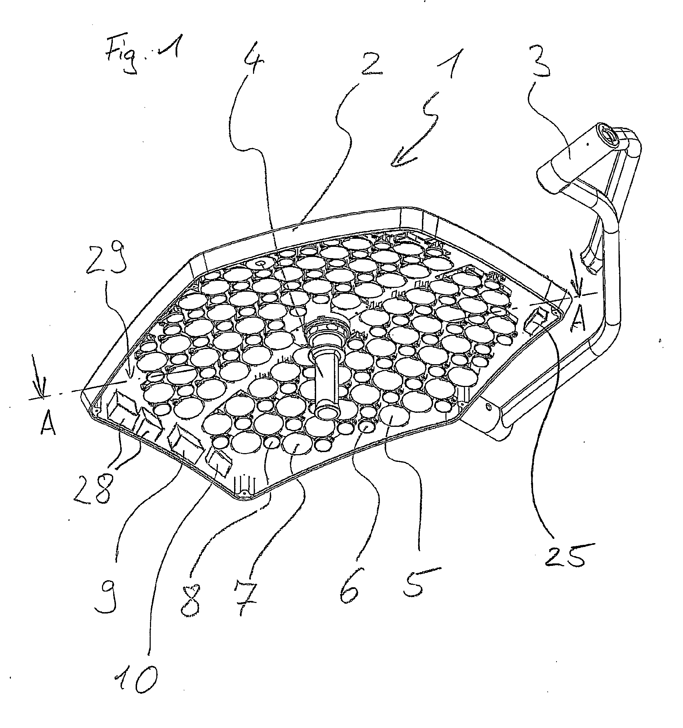

Fig. 1 zeigt eine Operationsleuchte 1, die einen Leuchtenkörper 2 und ein Tragsystem 3 aufweist, wobei von dem Tragsystem 3 nur ein so genannter Komfortbügel und ein Teil eines so genannten Viertelbügels gezeigt sind. An dem Leuchtenkörper 2 ist hier im Zentrum des Leuchtenkörpers 2 ein Handgriff 4 angeordnet. Der Handgriff 4 kann in alternativen Ausführungsformen auch an einer anderen Position an dem Leuchtenkörper 2 angeordnet sein. In dem Leuchtenkörper 2 sind erste Lichtquellen 5, zweite Lichtquellen 6, dritte Lichtquellen 7 und vierte Lichtquellen 8 angeordnet. Weiterhin nimmt der Leuchtenkörper 2 eine Steuerungsvorrichtung 9 auf, wobei die Steuerungsvorrichtung 9 in alternativen Ausführungsformen auch in einem separaten Gehäuse und/oder an einer anderen Position der Operationsleuchte 1 oder ihrer Umgebung vorgesehen sein kann.

Im Leuchtenkörper 2 ist außerdem ein Mittel 25 zum Auslösen der Änderung der individuellen Lichtstärken der Lichtquellen zum Anpassen eines nachstehend beschriebenen resultierenden Leuchtfelds, beispielsweise ein Bewegungssensor oder Beschleunigungssensor, vorgesehen, der mit der Steuerungsvorrichtung 9 verbunden ist. Mit dem Bewegungssensor wird eine Bewegung des Leuchtenkörpers 2 erfasst und nach Beendigung der Bewegung wird ein entsprechendes Signal an die Steuerungsvorrichtung 9 gegeben. Eine Bewegung kann aber auch alternativ auf eine andere Weise, beispielsweise durch eine Auswertung von Signalen einer Abstandsmessvorrichtung, erfasst werden. Nach Beendigung der Bewegung führt die Steuerungsvorrichtung 9 dann eine Anpassung des resultierenden Leuchtfelds durch. Alternativ kann das Auslösen auch manuell, beispielsweise durch Betätigen oder Loslassen eines Schalters oder Sensors, erfolgen.

Des Weiteren sind in dem Leuchtenkörper 2 Ansteuereinheiten 28 für jede Lichtquelle 5, 6, 7, 8 zum Ansteuern ihrer Lichtstärke vorgesehen.

Das Tragsystem 3 ermöglicht es, den Leuchtenkörper 2 innerhalb eines festgelegten räumlichen Bewegungsbereichs an einer beliebigen Position in einer beliebigen Orientierung zu positionieren, um eine Operationsstelle eines Patienten möglichst optimal auszuleuchten.

Die Lichtquellen 5, 6, 7, 8 enthalten LEDs mit einer optischen Vorrichtung, um die Lichtstrahlen der LEDs jeweils zu einem Lichtbündel zu bündeln. Um eine geeignete Farbtemperatur bei einer guten Farbwiedergabe zu erzielen, können LEDs mit verschiedenen weißen Farbtönen (warmweiß und kaltweiß) verwendet werden. Dadurch kann auch eine Farbtemperatur des von der Operationsleuchte 1 abgestrahlten Lichts eingestellt werden. Für einen größeren Einstellbereich der Farbtemperatur können in alternativen Operationsleuchten auch farbige LEDs verwendet werden. Alternativ können Lichtquellen, die Licht mit der gleichen Farbtemperatur abgeben, verwendet werden.

Die optischen Vorrichtungen sind hier Linsen, wobei zwei unterschiedliche Typen von Linsen eingesetzt werden, die das abgestrahlte Licht der LEDs so lenken, dass Lichtbündel erzeugt werden. Ein Linsentyp, hier ein Typ mit einem großen Linsendurchmesser in den Lichtquellen 5, 7, erzeugt ein Lichtbündel, das ein Leuchtfeld mit einem kleinen Durchmesser erzeugt, ein anderer Linsentyp, hier mit ein Typ mit einem kleinen Linsendurchmesser in den Lichtquellen 6, 8, erzeugt ein Lichtbündel, das ein Leuchtfeld mit einem großen Leuchtfelddurchmesser erzeugt. Alternativ ist auch die Verwendung von Linsen möglich, die den selben Durchmesser aufweisen aber unterschiedliche optische Eigenschaften haben. Die durch die verschiedenen Linsen erzeugten Leuchtfelder weisen auf Grund von unterschiedlichen optisch wirksamen Flächen der Linsen und/oder den unterschiedlichen Durchmessern der Linsen unterschiedliche Lichtverteilungen und unterschiedliche Leuchtfelddurchmesser auf. In weiteren alternativen Ausführungsformen ist der Einsatz von anderen Mitteln zur Erzeugung von Lichtbündeln, die Leuchtfelder mit verschiedenen Durchmessern erzeugen, wie z.B. Reflektoren, denkbar.

In einer ersten Ausführungsform sind nur die Lichtquellen 5, 6 vorgesehen, die in dem Leuchtenkörper 2 starr angebracht sind.

In einer zweiten Ausführungsform sind die Lichtquellen 5, 6 analog zu den später beschriebenen Lichtquellen 7, 8 kippbar gelagert und alternativ einzeln, in Gruppen oder gemeinsam mit einer Antriebsvorrichtung 10 zum Kippen versehen.

Wie später beschrieben, ist es zur Erzeugung von verschiedenen Leuchtfelddurchmessern in verschiedenen Abständen zum Leuchtenkörper 2 günstig, aber nicht zwingend, in einer dritten Ausführungsform zusätzlich zu den starren Lichtquellen weitere Lichtquellen vorzusehen, die kippbar in dem Leuchtenkörper 2 angeordnet sind. Hierfür sind die Lichtquellen 7, 8 vorgesehen, die entsprechend kippbar gelagert sind, und für die in dem Leuchtenkörper 2 die Antriebsvorrichtung 10 zum Kippen der Lichtquellen 7, 8 alternativ einzeln, in Gruppen oder gemeinsam vorgesehen ist. Die Lichtquellen 7 entsprechen bis auf die Kippbarkeit den Lichtquellen 5, und die Lichtquellen 8 entsprechen bis auf die Kippbarkeit den Lichtquellen 6.

Die Lichtquellen 5, 6 sind so angeordnet, dass die Lichtaustrittsflächen sämtlicher Linsen in einer sphärischen Fläche mit einem Radius von 1300 mm angeordnet sind. In alternativen Ausführungsformen können die Lichtquellen auch so angeordnet sein, dass die Lichtaustrittsflächen nicht in einer Fläche angeordnet sind, dass die Fläche nicht sphärisch ist, oder die sphärische Fläche einen anderen Radius aufweist.

Die Lichtquellen 5, 6, 7, 8 sind jeweils über die Ansteuereinheiten 28 mit der Steuerungsvorrichtung 9 verbunden, und die Lichtstärken der einzelnen Lichtquellen 5, 6, 7, 8 werden von der Steuerungsvorrichtung 9 angesteuert. Die Antriebsvorrichtung 10 zum Kippen der Lichtquellen 7 und 8 ist ebenfalls mit der Steuerungsvorrichtung 9 verbunden und ein entsprechender durch die Antriebsvorrichtung 10 einstellbarer Kippwinkel der Lichtquellen 7, 8 ist durch die Steuerungsvorrichtung 9 ansteuerbar.

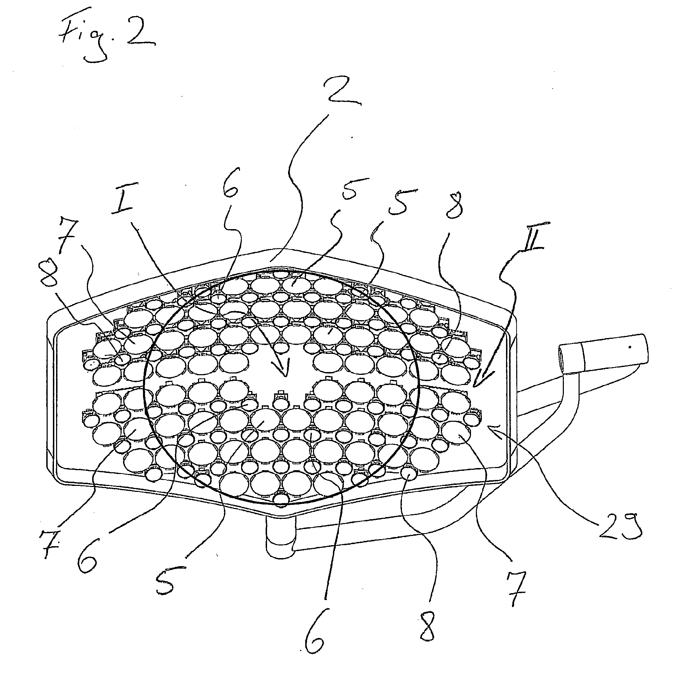

Fig. 2 zeigt den Leuchtenkörper 2 der dritten Ausführungsform, ohne Handgriff dargestellt, von schräg unten. Hierbei ist zu erkennen, dass der Leuchtenkörper 2 eine Lichtaustrittsfläche 29 aufweist, und die Lichtaustrittsfläche 29 in einen kreisförmigen inneren Bereich I und einen darum herum angeordneten äußeren Bereich II unterteilt ist. Die Lichtquellen 5, 6, die sich zumindest in dem Bereich I befinden, sind starr angeordnet, und die Lichtquellen 7, 8, die sich zumindest im Bereich II befinden, also einen größeren Abstand zu einer später gezeigten optischen Achse des Leuchtenkörpers 2 haben, sind kippbar in dem Leuchtenkörper 2 befestigt.

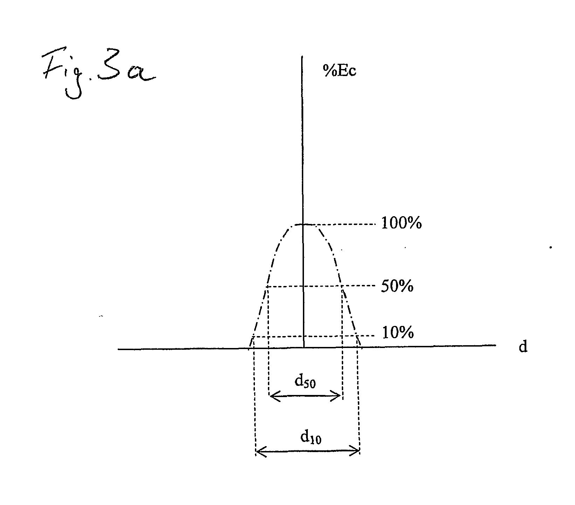

In den Figuren 3a bis 3d wird das Prinzip der Veränderung des Leuchtfelddurchmessers durch unterschiedliches Ansteuern der Lichtquellen erklärt.

Unter Leuchtfeld versteht man den beleuchteten Bereich auf einer Operationsstelle. In Fig. 3a ist beispielsweise in einem Diagramm eine normgerechte Lichtverteilung in dem Leuchtfeld einer Operationsleuchte gezeigt. Die Lichtverteilung im Leuchtfeld ist gemäß der derzeit geltenden Norm DIN EN 60601-2-41:2010 wie folgt definiert: Das Leuchtfeld weist eine zentrale Beleuchtungsstärke von 100% E_{c} auf. Der Durchmesser eines Kreises um das Leuchtfeldzentrum, auf dem die Beleuchtungsstärke 10% der zentralen Beleuchtungsstärke E_{c} beträgt, ist der Leuchtfelddurchmesser. Dieser Leuchtfelddurchmesser wird als "d₁₀" bezeichnet. Ein weiterer Kennwert des Leuchtfelds ist ein Durchmesser, bei dem die Beleuchtungsstärke 50% der zentralen Beleuchtungsstärke E_{c} beträgt. Dieser Durchmesser wird auch als "d₅₀" bezeichnet. Der Durchmesser d₅₀ ist gemäß der Norm so festgelegt, dass er mindestens die Hälfte des Leuchtfelddurchmessers d₁₀ betragen muss. Diese Bedingungen müssen bei einem Abstand von 1000 mm zwischen der Lichtaustrittsfläche und dem Leuchtfeld eingehalten werden.

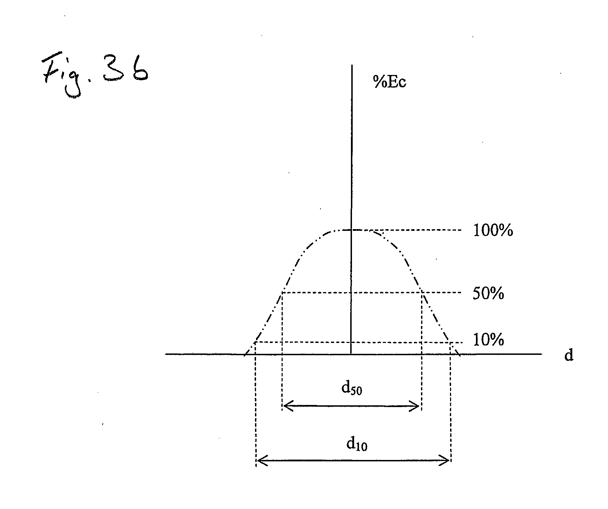

In Fig. 3a ist mit einer Strich-Punkt-Linie die Lichtverteilung einer Lichtquelle gezeigt, die prinzipiell der Lichtquelle 5 in den Ausführungsbeispielen entspricht. In Fig. 3b ist mit einer Strich-Zweipunkt-Linie die Lichtverteilung einer Lichtquelle gezeigt, die prinzipiell der Lichtquelle 6 in den Ausführungsbeispielen entspricht. Der Leuchtfelddurchmesser d₁₀ der Lichtquelle 5 (Fig. 3a) ist kleiner als der Leuchtfelddurchmesser d₁₀ der Lichtquelle 6 (Fig. 3b).

Als gestrichelte horizontale Linien sind in den Figuren 3a und 3b die relativen Beleuchtungsstärken 10%, 50% und 100% der jeweiligen Lichtquellen eingetragen. Aus den Schnittpunkten der gestrichelten horizontalen Linien und der Linie, die die Lichtverteilung angibt, ergeben sich der Durchmesser d₅₀ und der Leuchtfelddurchmesser d₁₀. Wie aus den Diagrammen zu erkennen ist, erfüllen die Leuchtfelder die Normenanforderung hinsichtlich der Lichtverteilung, da der Durchmesser d₅₀ mehr als die Hälfte des Leuchtfelddurchmessers d₁₀ beträgt.

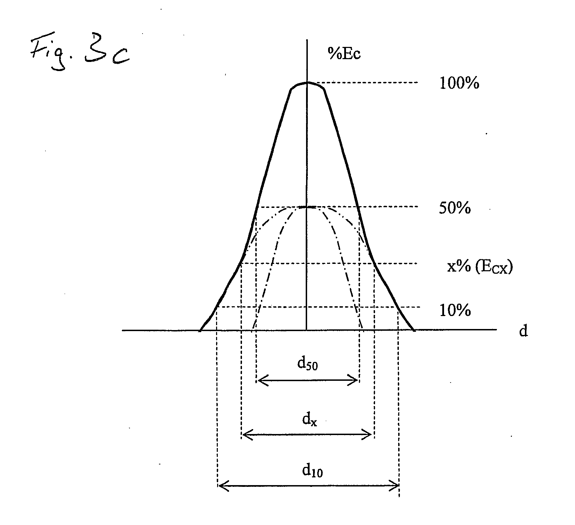

In Fig. 3c ist mit den identischen Linientypen jeweils die Lichtverteilung aus den Fig. 3a und 3b übertragen und mit einer durchgezogenen Linie ist die Lichtverteilung in einem resultierenden Leuchtfeld gezeigt, das von den beiden Lichtquellen aus Fig. 3a und Fig. 3b durch Überlagerung des Lichts gebildet wird. Durch die Überlagerung addieren sich die jeweiligen Beleuchtungsstärken auf den identischen Durchmessern.

In der in der Fig. 3c gezeigten Situation ist die zentrale Beleuchtungsstärke E_{c} der einzelnen Lichtquellen gleich groß und beträgt deshalb jeweils 50% der zentralen Beleuchtungsstärke E_{c} des resultierenden Leuchtfelds. Als gestrichelte horizontale Linien sind hier die Beleuchtungsstärken eingetragen, die 10% bzw. 50% bzw. 100% der zentralen Beleuchtungsstärke E_{c} des resultierenden Leuchtfelds betragen. Anhand der Schnittpunkte dieser horizontalen Linien mit der Linie der Lichtverteilung des resultierenden Leuchtfelds ergeben sich jeweils die Durchmesser, auf denen die Beleuchtungsstärken 10% bzw. 50% der zentralen Beleuchtungsstärke E_{c} des resultierenden Leuchtfelds betragen.

Darüber hinaus ist in der Fig. 3c eine relative Beleuchtungsstärke x% der zentralen Beleuchtungsstärke E_{c} eingetragen, die beliebig entweder werksseitig oder vom Nutzer festgelegt und voreingestellt werden kann. Hierbei ergibt sich dann durch die Schnittpunkte mit der Linie der Beleuchtungsstärke des resultierenden Leuchtfelds der Durchmesser dₓ eines Kreises, auf dem die Beleuchtungsstärke x% der zentralen Beleuchtungsstärke E_{c} des resultierenden Leuchtfelds beträgt. Der Durchmesser dₓ kann, wie später beschrieben, als ein Durchmesser, auf dem eine beliebige voreingestellte relative Beleuchtungsstärke E_{cx} des resultierenden Leuchtfelds vorliegt, konstant gehalten werden. Üblich ist, dass der Leuchtfelddurchmesser d₁₀ als der Durchmesser dₓ festgelegt wird, der bei einer Veränderung des Abstands unverändert bleiben soll.

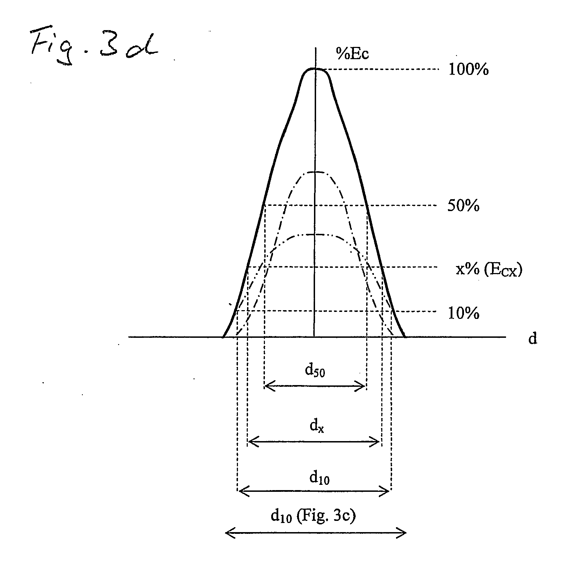

Fig. 3d zeigt die Lichtverteilung, wenn die einzelnen Lichtquellen so angesteuert werden, dass unterschiedliche zentrale Beleuchtungsstärken E_{c} der einzelnen Lichtquellen vorliegen. In diesem Fall ist die zentrale Beleuchtungsstärke E_{c} der Lichtquelle, die das Leuchtfeld mit dem kleineren Leuchtfelddurchmesser erzeugt (Strich-Zweipunkt-Linie; Fig. 3b) geringer als in der in Fig. 3c gezeigten Situation. Die zentrale Beleuchtungsstärke E_{c} der Lichtquelle, die das Leuchtfeld mit dem größeren Durchmesser erzeugt (Strich-Punkt-Linie; Fig. 3a) ist größer als in der in Fig. 3c gezeigten Situation.

Wie aus den Diagrammen der Fig. 3c und 3d zu erkennen ist, erfüllen auch die resultierenden Leuchtfelder die Normenanforderung hinsichtlich der Lichtverteilung, da der Durchmesser d₅₀ mehr als die Hälfte des Leuchtfelddurchmessers d₁₀ beträgt.

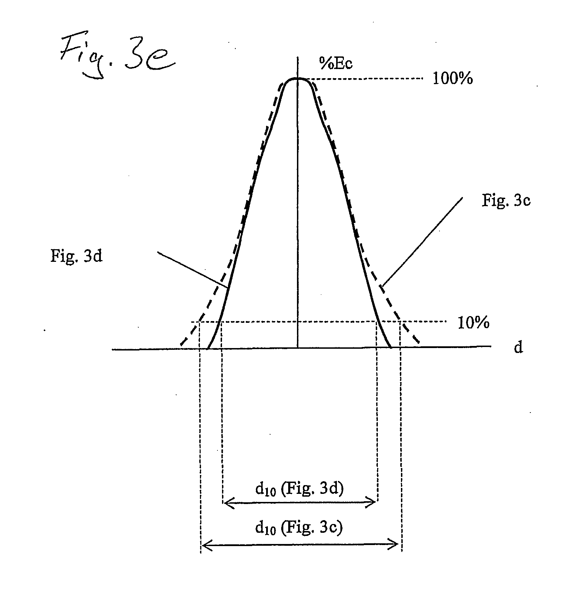

Anhand des in Fig. 3e gezeigten Vergleichs der Leuchtfelddurchmesser d₁₀ der resultierenden Leuchtfelder aus Fig. 3c und Fig. 3d lässt sich erkennen, dass bei einer Erhöhung der Beleuchtungsstärke durch die Lichtquelle, die einen kleineren Leuchtfelddurchmesser d₁₀ erzeugt, und einer Verringerung der Beleuchtungsstärke durch die Lichtquelle, die einen größeren Leuchtfelddurchmesser d₁₀ erzeugt, der Leuchtfelddurchmesser d₁₀ des resultierenden Leuchtfelds kleiner wird.

Grundsätzlich wird ein kleinerer Leuchtfelddurchmesser d₁₀ erzeugt, wenn die Beleuchtungsstärke der Lichtquelle, die einen kleineren Leuchtfelddurchmesser d₁₀ erzeugt, erhöht wird und/oder die Beleuchtungsstärke der Lichtquelle, die einen größeren Leuchtfelddurchmesser d₁₀ erzeugt, verringert wird.

Analog wird ein größerer Leuchtfelddurchmesser d₁₀ erzeugt, wenn die Beleuchtungsstärke der Lichtquelle, die einen kleineren Leuchtfelddurchmesser d₁₀ erzeugt, verringert wird und/oder die Beleuchtungsstärke der Lichtquelle, die einen größeren Leuchtfelddurchmesser d₁₀ erzeugt, erhöht wird.

Um die zentrale Beleuchtungsstärke E_{c} des resultierenden Leuchtfelds konstant zu halten, kann die zentrale Beleuchtungsstärke E_{c} einer der Lichtquellen um den gleichen Betrag verringert werden, um den die zentrale Beleuchtungsstärke E_{c} der anderen Lichtquelle erhöht wird, so dass die Summe der einzelnen zentralen Beleuchtungsstärken E_{c} gleich bleibt.

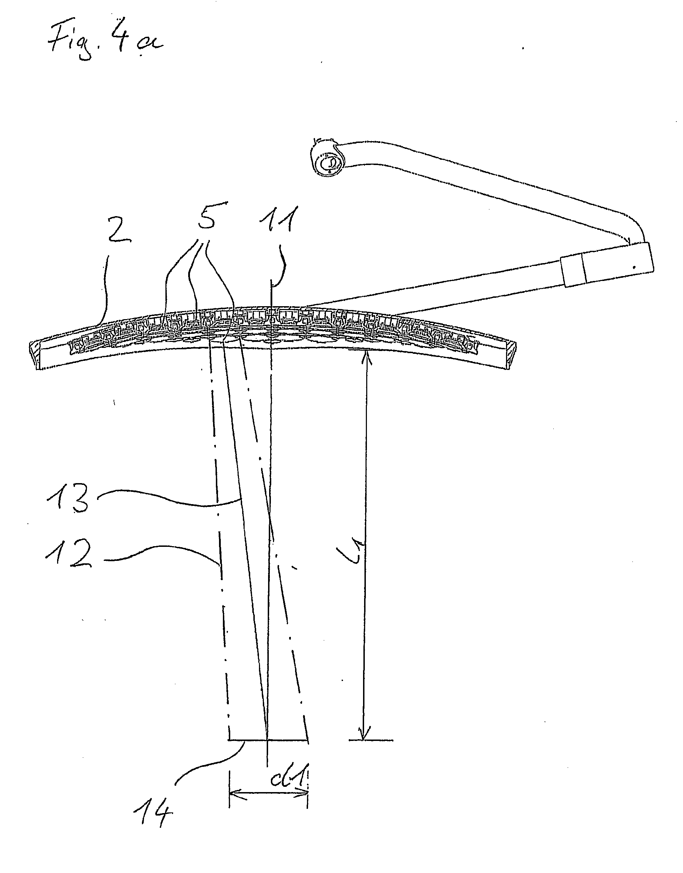

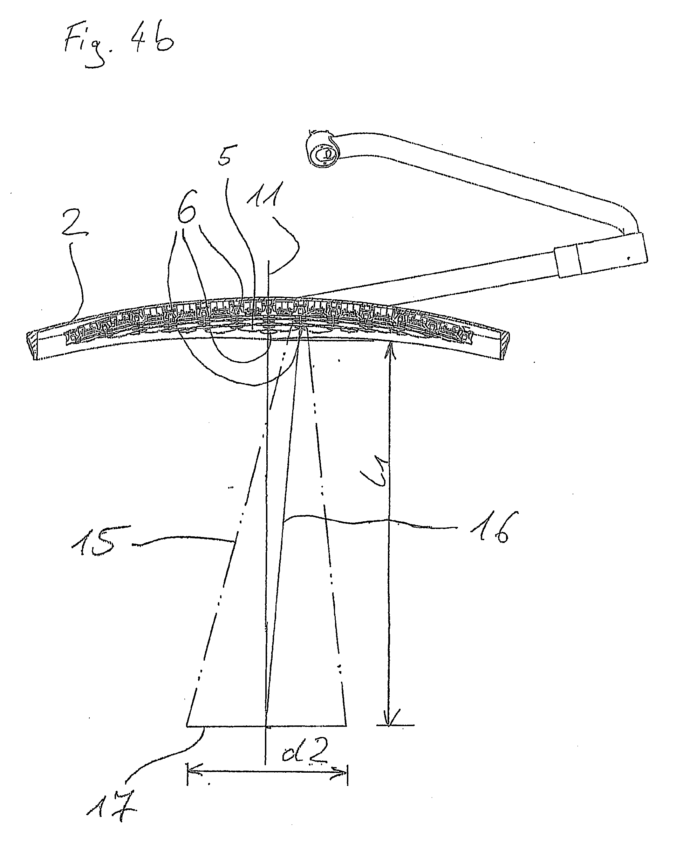

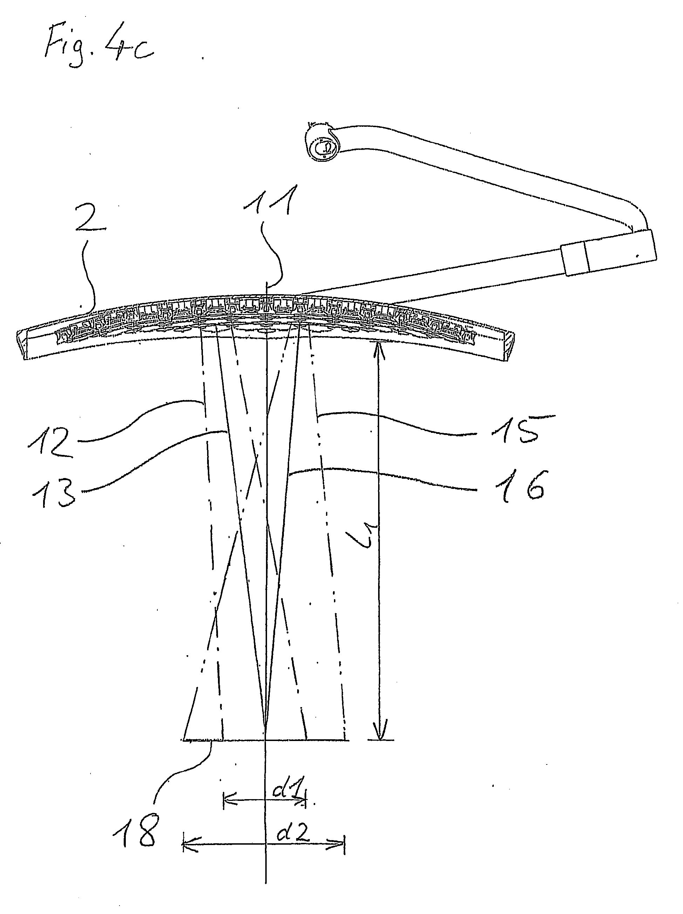

In den Fig. 4a bis 4d wird prinzipiell gezeigt, wie Leuchtfelder durch Überlagerung von verschiedenen Lichtbündeln erzeugt werden. Die Darstellung der verschiedenen Lichtbündel ist nicht so zu verstehen, dass die Darstellung eine exakte Hell-Dunkel-Grenze darstellt. Auch die Darstellung von verschiedenen Durchmessern stellt keine exakte Hell-Dunkel-Grenze dar, da bei der Definition des Durchmessers als Leuchtfelddurchmesser d₁₀ (Fig. 4a, 4b, 4c) auch außerhalb dieses Durchmessers Licht mit einem Anteil von kleiner als 10% der zentralen Beleuchtungsstärke E_{c} (Streulicht) vorhanden sein kann.

In Fig. 4a ist eine Schnittdarstellung des Leuchtenkörpers 2 entlang einer Schnittlinie A-A in Fig. 1 gezeigt. Der Leuchtenkörper 2 weist eine optische Achse 11 auf, auf der sich der Mittelpunkt eines erzeugten Leuchtfelds befindet.

Die Lichtquellen 5 strahlen jeweils ein Lichtbündel 12 ab, das durch eine Strichpunktlinie dargestellt ist, wobei hier aus Gründen der Übersichtlichkeit nur eines der Lichtbündel 12 gezeigt ist. Das Lichtbündel 12 hat eine Achse 13, die sich in einem Abstand l₁ von dem Leuchtenkörper 2 mit dessen optischer Achse 11 schneidet. Die weiteren Lichtquellen 5 sind in dem Leuchtenkörper 2 so verteilt angeordnet, dass die Achsen ihrer Lichtbündel die optische Achse 11 in dem selben Punkt wie die Achse 13 der Lichtquelle 5 schneiden, so dass sämtliche Lichtquellen 5 ein Leuchtfeld 14 bilden.

Der Abstand l₁ ist in dieser Ausführungsform auf 1300 mm festgelegt, kann aber in anderen Ausführungsformen abhängig von dem Verwendungszweck und der Leuchtenkörpergröße auf einen anderen Wert festgelegt werden.

Im Abstand l₁ von dem Leuchtenkörper 2 wird das erste Leuchtfeld 14 auf der Operationsstelle gebildet. Das erste Leuchtfeld 14, das durch die Lichtbündel der ersten Lichtquellen 5 gebildet wird, weist einen Leuchtfelddurchmesser d₁₀ auf, der mit d1 bezeichnet ist.

Auch in Fig. 4b ist eine Schnittdarstellung des Leuchtenkörpers 2 entlang der Schnittlinie A-A in Fig. 1 gezeigt. Der Leuchtenkörper 2 weist auch hier die optische Achse 11 auf.

Die Lichtquellen 6 strahlen jeweils ein Lichtbündel 15 ab, das durch eine Strich-Zweipunkt-Linie dargestellt ist, wobei auch hier aus Gründen der Übersichtlichkeit auch nur eines der Lichtbündel 15 gezeigt ist. Das Lichtbündel 15 hat eine Achse 16, die sich in dem Abstand l₁ von dem Leuchtenkörper 2 mit dessen optischer Achse 11 schneidet. Die weiteren Lichtquellen 6 sind in dem Leuchtenkörper 2 so verteilt angeordnet, dass die Achsen ihrer Lichtbündel die optische Achse 11 in dem selben Punkt wie die Achse 16 der gezeigten Lichtquelle 6 schneiden, so dass sämtliche Lichtquellen 6 ein zweites Leuchtfeld 17 bilden.

Der Abstand l₁ ist auch hier auf 1300 mm festgelegt, kann aber in anderen Ausführungsformen abhängig von dem Verwendungszweck und der Leuchtenkörpergröße auf einen anderen Wert bestimmt werden.

Im Abstand l₁ von dem Leuchtenkörper 2 wird durch die Lichtbündel 15 der zweiten Lichtquellen 6 das zweite Leuchtfeld 17 auf der Operationsstelle gebildet. Das zweite Leuchtfeld 17 weist einen Leuchtfelddurchmesser d₁₀ auf, der mit d2 bezeichnet ist.

Der Durchmesser d2 des durch die zweiten Lichtquellen 6 gebildeten zweiten Leuchtfelds 17 ist größer als der Durchmesser d1 des durch die ersten Lichtquellen 5 gebildeten ersten Leuchtfelds 14 (siehe Fig. 4a).

Fig. 4c zeigt die überlagerten Lichtbündel 12 und 15, die in den Figuren 4a und 4b beschrieben wurden.

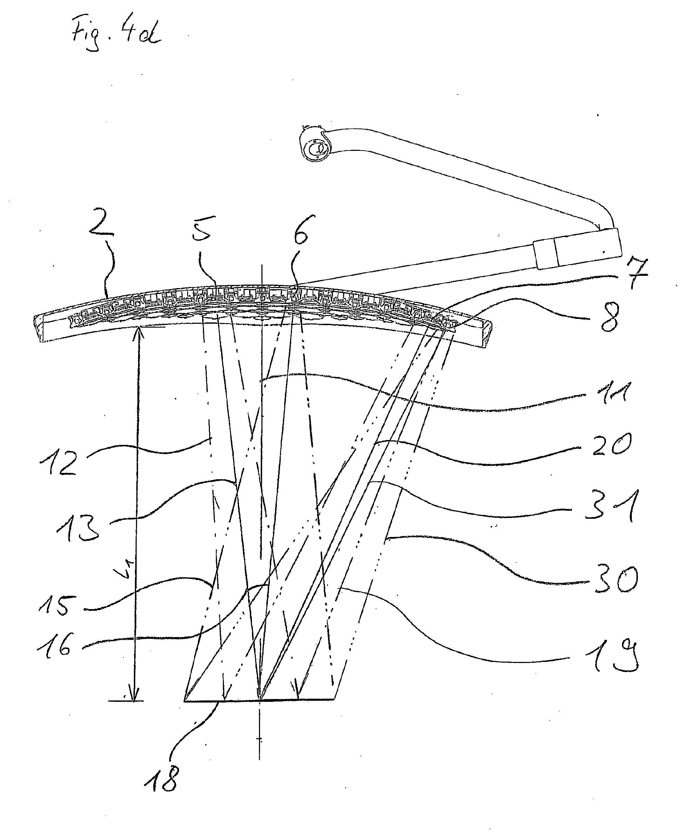

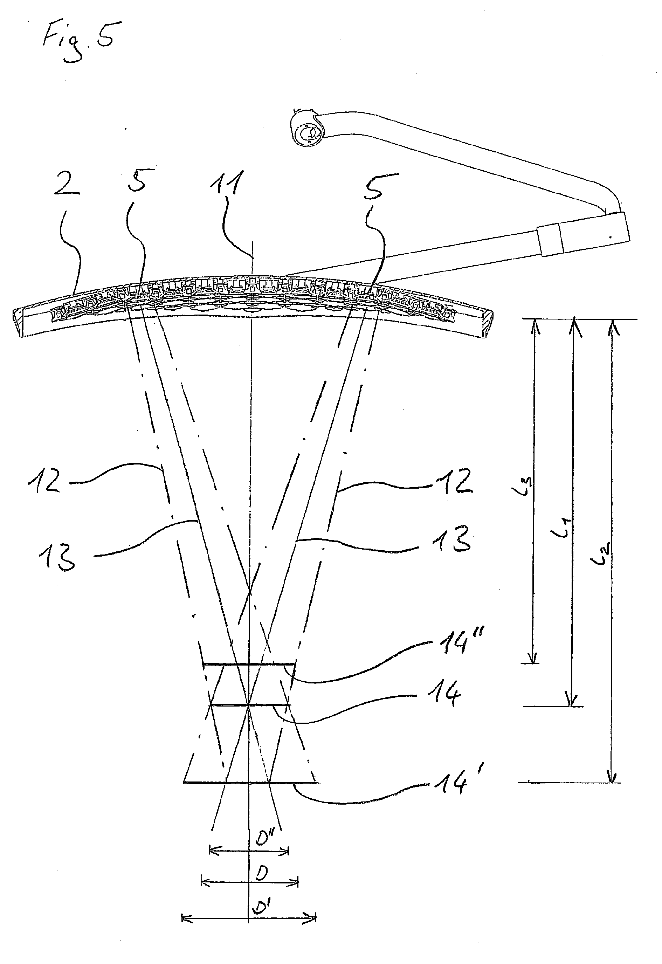

Durch die Überlagerung der Lichtbündel 12, 15 bilden die erste Lichtquelle 5 und die zweite Lichtquelle 6 auf der Operationsstelle, die sich in dem Abstand l₁ von dem Leuchtenkörper 2 befindet, auf die zu den Fig. 3c, 3d und 5 beschriebene Art und Weise, ein resultierendes Leuchtfeld 18. Das Leuchtfeld mit dem kleineren Durchmesser d₁, das durch das Lichtbündel 12 gebildet wird, und das Leuchtfeld mit dem größeren Durchmesser d2, das durch das Lichtbündel 15 gebildet wird, werden dabei überlagert. In Fig. 4d ist ein exemplarisches optionales drittes Lichtbündel 19 gezeigt, das mit einer Strich-Drei-Punkt-Linie dargestellt ist. Das dritte Lichtbündel 19 wird von der dritten Lichtquelle 7 ausgestrahlt, wobei auch hier aus Gründen der Übersichtlichkeit nur eines der dritten Lichtbündel 19 gezeigt ist. Die weiteren Lichtquellen 7 sind in dem Leuchtenkörper 2 so angeordnet, dass die Achsen ihrer Lichtbündel die optische Achse 11 in dem selben Punkt wie eine Achse 20 der gezeigten Lichtquelle 7 schneiden. Ein exemplarisches optionales viertes Lichtbündel 30, das mit einer Strich-Vier-Punkt-Linie dargestellt ist, wird von der vierten Lichtquelle 8 ausgestrahlt, wobei auch hier aus Gründen der Übersichtlichkeit nur eines der vierten Lichtbündel 30 gezeigt ist. Die weiteren vierten Lichtquellen 8 sind in dem Leuchtenkörper 2 so angeordnet, dass die Achsen ihrer Lichtbündel die optische Achse 11 in dem selben Punkt wie eine Achse 31 der Lichtquelle 8 schneiden. Das dritte Lichtbündel 19 weist die Achse 20 auf, und das vierte Lichtbündel 30 weist die Achse 31 auf, die sich mit der optischen Achse 11 des Leuchtenkörpers 2 bei dieser Einstellung des Kippwinkels der dritten und vierten Lichtquellen 7, 8 in dem selben Schnittpunkt schneiden, wie die Achsen 13 der Lichtbündel 12 der ersten Lichtquellen 5, und die Achsen 16 der Lichtbündel 15 der zweiten Lichtquellen 6, hier im Abstand l₁. Das dritte Lichtbündel 19 erzeugt ein Leuchtfeld mit einem kleineren Durchmesser und das vierte Lichtbündel 30 erzeugt ein Leuchtfeld mit einem größeren Durchmesser und gemeinsam mit dem ersten Lichtbündel 12 und dem zweiten Lichtbündel 15 erzeugen das dritte Lichtbündel 19 und das vierte Lichtbündel 20 auf die zu den Fig. 3c, 3d und 5 beschriebene Art und Weise das resultierende Leuchtfeld 18. Die Leuchtfelder mit den kleineren Durchmessern, die durch die Lichtbündel 12 und 19 gebildet werden, und die Leuchtfelder mit dem größeren Durchmesser, die durch die Lichtbündel 15 und 30 gebildet werden, werden dabei überlagert.

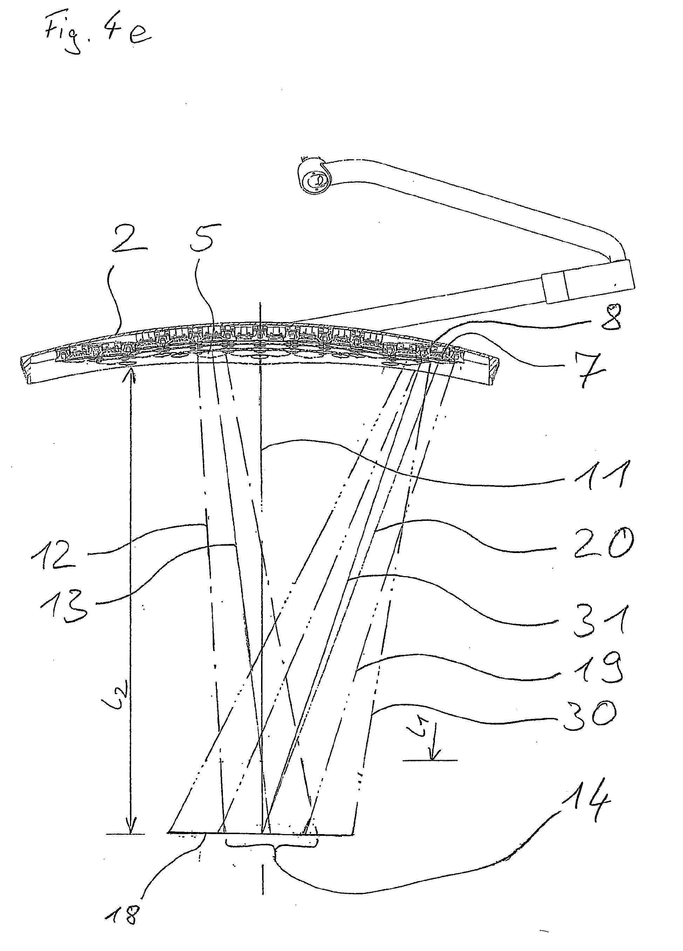

Fig. 4e zeigt eine Situation, in der der Abstand der Operationsstelle, auf der ein Leuchtfeld gebildet wird, größer, nämlich l₂, ist. Daher werden die Leuchtfelder im Abstand l₂ abgebildet.

Das gezeigte Lichtbündel 12 von einer der ersten Lichtquellen 5 wird dann in dem ersten Leuchtfeld 14 abgebildet, das nicht konzentrisch zu der optischen Achse 11 ist, da sich seine Achse 13 im Abstand l₁ mit der optischen Achse 11 schneidet und sich die Achse 13 im Abstand l₂ nicht mit der optischen Achse 11 schneidet. Durch die Überlagerung von mehreren Lichtbündeln 12, ausgehend von über den Umfang des Leuchtenkörpers 2 verteilten Lichtquellen 5, entsteht jedoch wieder ein Leuchtfeld aus den Lichtbündeln 12 der ersten Lichtquellen 5, das konzentrisch zu der optischen Achse 11 ist.

Die dritten Lichtquellen 7 und die vierten Lichtquellen 8 werden mit Hilfe der Steuerungsvorrichtung 9, die die Antriebsvorrichtung 10 ansteuert, so gekippt, dass sich die Achsen 20, 31 der Lichtbündel 19, 30 in dem Abstand l₂ auf der optischen Achse 11 schneiden. Nach einer Veränderung des Abstands durch Bewegen des Leuchtenkörpers 2 werden die dritten Lichtquellen 7 und die vierten Lichtquellen 8 durch die Antriebsvorrichtung 10 so gekippt, dass sich der Schnittpunkt der Achsen 20 und 31 jeweils in dem tatsächlichen Abstand der Operationsstelle von dem Leuchtenkörper 2 befindet.

In Fig. 5 ist eine Schnittdarstellung des Leuchtenkörpers 2 mit einer prinzipiellen Darstellung von drei Leuchtfeldern 14, 14', 14'', die von zwei der ersten Lichtquellen 5 erzeugt werden. Wie aus Fig. 5 zu ersehen ist, wird das Leuchtfeld 14 auf einer Operationsstelle im Abstand l₁, das Leuchtfeld 14' auf einer Operationsstelle im Abstand l₂ und das Leuchtfeld 14'' auf einer Operationsstelle im Abstand l₃ erzeugt.

Der Leuchtenkörper 2 weist auch hier die optische Achse 11 auf, auf der sich die Mittelpunkte der erzeugten Leuchtfelder befinden. Die Lichtquellen 5 strahlen jeweils das Lichtbündel 12 ab, wobei hier aus Gründen der Übersichtlichkeit nur zwei der Lichtbündel 12 gezeigt sind. Die Lichtbündel 12 haben auch hier jeweils die Achse 13, die sich in dem Abstand l₁ von dem Leuchtenkörper 2 mit dessen optischer Achse 11 schneiden. Die weiteren Lichtquellen 5 sind in dem Leuchtenkörper 2 so verteilt angeordnet, dass die Achsen ihrer Lichtbündel die optische Achse 11 in dem selben Punkt wie die Achse 13 der Lichtquelle 5 schneiden, so dass sämtliche Lichtquellen 5 das Leuchtfeld 14 auf der Operationsstelle im Abstand l₁ bilden.

Aus der Fig. 5 ist prinzipiell ersichtlich, dass die Durchmesser D, D', D'' abhängig von den Abständen l₁, l₂ und l₃ der Leuchtfelder 14, 14', 14'' von dem Leuchtenkörper 2 unterschiedlich groß sind. Das Leuchtfeld 14, das auf der Operationsstelle im Abstand l₁, in dem sich die Achsen 13 mit der optischen Achse 11 schneiden, gebildet wird, weist den kleinsten Durchmesser D auf. Bei einer Veränderung des Abstands der Operationsstelle beispielsweise auf l₂ oder l₃ werden die Leuchtfelder 14', 14'' erzeugt, die die größeren Durchmesser D', D" aufweisen. Die Durchmesser D, D', D" sind nur prinzipiell dargestellt und stellen nicht zwingend die Leuchtfelddurchmesser d₁₀ oder die Durchmesser dₓ, bei dem eine relative Beleuchtungsstärke E_{cx} vorliegt, der Leuchtfelder 14, 14', 14" dar. Durch diese Darstellung wird lediglich gezeigt, dass sich prinzipiell der Durchmesser eines Leuchtfelds ändert, wenn der Abstand zwischen dem Leuchtenkörper und der Operationsstelle verändert wird.

Wie die prinzipiell dargestellten Durchmesser D, D', D" der Leuchtfelder 14, 14', 14'' ändern sich bei einer Abstandsänderung auch sowohl der Leuchtfelddurchmesser d₁₀ als auch der Durchmesser dₓ, bei dem eine relative Beleuchtungsstärke E_{cx} vorliegt.

Um bei einer Veränderung des Abstands von l₁ auf l₂ oder l₃ die Vergrößerung des Leuchtfelddurchmessers d₁₀ oder des Durchmessers dₓ, bei dem die relative Beleuchtungsstärke E_{cx} vorliegt, zu verhindern, ist es erforderlich, die Lichtquelle 5, die das Leuchtfeld mit dem kleineren Leuchtfelddurchmesser d1 erzeugt (Fig. 4a, 4c), so anzusteuern, dass dessen Lichtstärke erhöht wird und/oder die Lichtquelle 6, die das Leuchtfeld mit dem größeren Leuchtfelddurchmesser d2 erzeugt (Fig. 4b, 4c), so anzusteuern, dass dessen Lichtstärke verringert wird.

Umgekehrt ist es bei einer Abstandsänderung von den Abständen l₂ oder l₃ auf l₁ erforderlich, die Verkleinerung des Leuchtfelddurchmessers d₁₀ oder des Durchmessers dₓ, bei dem die relative Beleuchtungsstärke E_{cx} vorliegt, zu verhindern. Dazu ist es erforderlich, die Lichtquelle 5, das das Leuchtfeld mit dem kleineren Leuchtfelddurchmesser d1 erzeugt (Fig. 4a, 4c), so anzusteuern, dass dessen Lichtstärke verringert wird und/oder die Lichtquelle 6, die das Leuchtfeld mit dem größeren Leuchtfelddurchmesser d2 erzeugt (Fig. 4b, 4c), so anzusteuern, dass dessen Lichtstärke erhöht wird.

Nach der Ansteuerung der Lichtquellen 5, 6, so dass eine Veränderung des Leuchtfelddurchmessers d₁₀ verhindert wird, wird dann gegebenenfalls die Ansteuerung der Lichtquellen so angepasst, dass wieder die vorherige zentrale Beleuchtungsstärke E_{c} vorliegt.

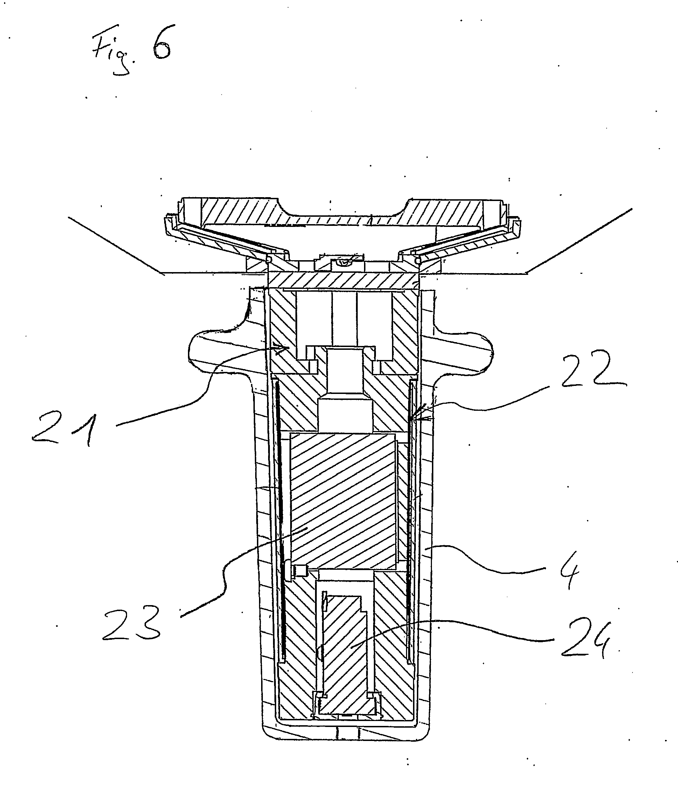

In Fig. 6 ist der Handgriff 4 der Operationsleuchte 1 gezeigt. Der Handgriff 4 ist über eine Handgriffaufnahme 21 geschoben und ist mit Hilfe eines nicht gezeigten Rastmechanismus befestigt. An der Handgriffaufnahme 21 ist eine Bedieneinrichtung mit zumindest einem Sensor 22 als Eingabemittel, und einer Auswerteeinheit 23 vorgesehen, die ein berührungsloses Bedienen der Operationsleuchte 1 ermöglicht.

Berührungslos heißt in diesem Zusammenhang, dass zwar die Bedieneinrichtung selbst, insbesondere der Sensor 22 der Bedieneinrichtung, nicht berührt wird, sterile Bauteile des Leuchtenkörpers, wie hier der Griff 4, die den Sensor der Bedieneinrichtung abdecken, jedoch berührt werden.

Mit der Bedieneinrichtung können der gewünschte Durchmesser dₓ und die gewünschte zentrale Beleuchtungsstärke E_{c} eingestellt werden. Das Einstellen erfolgt beispielsweise über ein Entlangfahren eines Objekts (z.B. der Finger des Operateurs) an dem Handgriff 4 in axialer Richtung für eine Einstellung beispielsweise des Durchmessers dₓ und Entlangfahren am Umfang des Handgriffs 4 für beispielsweise die Einstellung der zentralen Beleuchtungsstärke E_{c}.

Es können jedoch in anderen Ausführungsformen auch andere Einstellelemente, wie z.B. Druck- oder Drehschalter, Drehregler mit sterilem Bedienknopf, o.ä. vorgesehen sein, mit denen der gewünschte Durchmesser dₓ eingestellt werden kann oder alternativ voreingestellte Durchmesser dₓ ausgewählt werden können. Es werden hierbei die gewünschten Werte stufenlos eingestellt, oder alternativ aus vorher festgelegten Durchmessern dₓ oder vorher festgelegten zentralen Beleuchtungsstärken E_{c} ausgewählt.

In der Handgriffaufnahme 21 ist weiterhin eine Vorrichtung 24 zum Erfassen des Abstands zwischen dem Leuchtenkörper 2 und der Operationsstelle, hier in Form eines Abstandssensors, der als ein Lasersensor ausgebildet ist, vorgesehen. Mit dem Lasersensor wird der Abstand zwischen dem Leuchtenkörper 2 und der zu beleuchtenden Operationsstelle gemessen. Alternativ können auch andere Arten von Abstandsmessvorrichtungen, z.B. Ultraschallsensoren, oder z.B. Winkelaufnehmer im Tragsystem 3 zur Bestimmung der Position des Leuchtenkörpers 2, vorgesehen sein.

Die erforderlichen Werte zur Ansteuerung der Lichtstärke der einzelnen Lichtquellen 5, 6, 7, 8 und zur Ansteuerung der Antriebsvorrichtung 10 zum Einstellen des Kippwinkels für die dritten und vierten Lichtquellen 7, 8 werden in Abhängigkeit von dem gewünschten Durchmesser dₓ (Leuchtfelddurchmesser d₁₀), der zentralen Beleuchtungsstärke E_{c} der Operationsleuchte 1 und des Arbeitsabstands zwischen dem Leuchtenkörper 2 und dem zu beleuchtenden Objekt empirisch ermittelt und in einem Kennfeld in einem Speicherbereich der Steuerungsvorrichtung 9 gespeichert. Die Werte können dahingehend ermittelt werden, dass neben dem gewünschten Durchmesser dₓ (Leuchtfelddurchmesser d₁₀) auch das erforderliche Verhältnis von d₅₀ und d₁₀ eingehalten wird. Alternativ kann auch eine Beziehung der verschiedenen Werte hinterlegt werden.

Die Lichtquellen 5, 6, 7, 8 können zu Gruppen zusammengefasst sein, die jeweils über eine Ansteuereinheit 28 angesteuert werden. Das Kriterium für die Gruppierung kann der Leuchtfelddurchmesser d₁₀ des erzeugten Leuchtfelds 14, 17 oder der Abstand der Lichtquelle 5, 6, 7, 8 von der optischen Achse 11, also u.a. die Zugehörigkeit zum Bereich I oder zum Bereich II, sein, wobei auch innerhalb der Bereiche weitere Gruppierungen, z.B. Gruppierung nach der Farbtemperatur der LEDs, möglich sind. Die Lichtquellen 5, 6, 7, 8 der einzelnen Gruppen sind dann mit jeweils einer Ansteuereinheit 28 verbunden und können in ihrer Lichtstärke unterschiedlich angesteuert werden.

Im Betrieb misst der Lasersensor die Entfernung zwischen dem Leuchtenkörper 2 und dem zu beleuchtenden Körper und die Operationsleuchte 1 wird durch die Steuerungsvorrichtung 9 auf Sollanfangswerte für den Durchmesser dₓ, bei dem die voreingestellte relative Beleuchtungsstärke E_{cx} des resultierenden Leuchtfelds 18 vorliegt, und die zentrale Beleuchtungsstärke E_{c} zur Erzeugung eines resultierenden Leuchtfelds 18 eingestellt.

Die Sollvorgaben für den Durchmesser dₓ, bei dem die voreingestellte relative Beleuchtungsstärke E_{cx} des resultierenden Leuchtfelds 18 vorliegt, und die zentrale Beleuchtungsstärke E_{c} können mit Hilfe der Bedieneinrichtung verändert bzw. eingestellt werden.

Die Steuerung steuert dann die Lichtstärke der Lichtquellen 5, 6, 7, 8 und die Antriebsvorrichtung 10 der kippbaren Lichtquellen 7, 8 so an, dass die gewünschte zentrale Beleuchtungsstärke E_{c} und der vorgegebene Durchmesser dₓ, bei dem die voreingestellte relative Beleuchtungsstärke E_{cx} des resultierenden Leuchtfelds 18 vorliegt, durch die Operationsleuchte 1 erzeugt werden. Entsprechend den Vorgaben für die zentrale Beleuchtungsstärke E_{c}, für den Durchmesser dₓ, bei dem die voreingestellte relative Beleuchtungsstärke E_{cx} des resultierenden Leuchtfelds 18 vorliegt, und dem gemessenen Abstand werden die Werte aus dem Speicherbereich abgerufen und als Betriebsdaten der Operationsleuchte 1 eingestellt.

Eine Positionsänderung des Leuchtenkörpers 2 oder eine Änderung von dessen Ausrichtung, also eine Veränderung des Arbeitsabstands, wird durch das Mittel 25 zum Auslösen der Änderung der individuellen Lichtstärken der Lichtquellen zum Anpassen des Leuchtfelds, hier den Bewegungssensor erfasst, und nach Beendigung der Bewegung wird der Abstand zwischen dem Leuchtenkörper 2 und dem zu beleuchtenden Körper durch den Lasersensor gemessen oder alternativ auf andere Art und Weise erfasst. Anhand dieses erfassten Werts werden der Durchmesser dₓ, bei dem die voreingestellte relative Beleuchtungsstärke E_{cx} des resultierenden Leuchtfelds 18 vorliegt, und die zentrale Beleuchtungsstärke E_{c} der Operationsleuchte 1 korrigiert, indem die nun zugehörigen Betriebsdaten aus dem Speicherbereich von der Steuerungsvorrichtung 9 abgerufen werden und die Lichtquellen 5, 6, 7, 8 in den einzelnen Gruppen durch die Steuerungsvorrichtung 9 mit einem entsprechenden Mischungsverhältnis der Bestromungsstärken angesteuert werden. Weiterhin wird die Antriebsvorrichtung 10 angesteuert, einen vorbestimmten Kippwinkel einzustellen. Durch diese Korrektur bleiben sowohl der Durchmesser dₓ des resultierenden Leuchtfelds 18 als auch, sofern entsprechend eingestellt, die zentrale Beleuchtungsstärke E_{c} auf dem Operationsfeld vor und nach der Bewegung unverändert.

Alternativ zu der Operationsleuchte 1, bei der sämtliche Lichtquellen 5, 6 in einem Leuchtenkörper 2 mit einem einzigen Gehäuse angeordnet sind, kann der Leuchtenkörper 2 auch mit mehreren Gehäusen, die als Module ausgebildet sind, vorgesehen sein, wobei dann jeweils eine Mehrzahl von starr angeordneten Lichtquellen 5, 6 vorgesehen ist, und die Lichtquellen 5, 6 miteinander in den verschiedenen Modulen angeordnet sind. Die Module können in dieser Ausführungsform zueinander kippbar sein, um die äußeren Lichtquellen so zu verkippen, dass sich ihre Achsen an gewünschten Punkten auf der optischen Achse des Leuchtenkörpers schneiden.

## Patentansprüche

1. Operationsleuchte (1) zum Ausleuchten einer in einem gewählten Abstand angeordneten Operationsstelle, aufweisend:
einen Leuchtenkörper (2) mit einer optischen Achse (11), der mindestens eine erste Lichtquelle (5) und eine zweite Lichtquelle (6) aufweist, wobei die erste Lichtquelle (5) ein Lichtbündel (12) abstrahlt, das eine erste Achse (13) hat, die sich in einem Abstand (1₁) von dem Leuchtenkörper (2) mit der optischen Achse (11) schneidet, die zweite Lichtquelle (6) ein Lichtbündel (15) abstrahlt, das eine zweite Achse (16) hat, die sich in dem selben Abstand (1₁) von dem Leuchtenkörper (2) mit dessen optischer Achse (11) schneidet, die erste Lichtquelle (5) ein erstes Leuchtfeld (14) und die zweite Lichtquelle (6) ein zweites Leuchtfeld (17) mit jeweils unterschiedlichen Durchmessern (d1, d2) auf der Operationsstelle bilden, und die Leuchtfelder (14, 17) ein resultierendes im Wesentlichen kreisförmiges Leuchtfeld (18) mit einer operationsleuchtennorm-gerechten Lichtverteilung, z.Zt. DIN EN 60601-2-41:2010, mit einer voreingestellten normgerechten relativen Beleuchtungsstärke (E_{cx}) bei einem vorbestimmten Durchmesser (dₓ) ergeben,
eine Steuerungsvorrichtung (9) für die Lichtquellen (5, 6),
Ansteuereinheiten (28), über die die Lichtquellen (5, 6) jeweils mit der Steuerungsvorrichtung (9) verbunde sind, wobei die Steuerungsvorrichtung (9) dazu angepasst ist, die Lichtstärke der ersten Lichtquelle (5) und die Lichtstärke der zweiten Lichtquelle (6) individuell anzusteuern, so dass bei dem gewählten Abstand die voreingestellte relative Beleuchtungsstärke (E_{cx}) bei dem vorbestimmten Durchmesser (dₓ) vorliegt,
eine Vorrichtung (24) zum Erfassen des Abstands zwischen dem Leuchtenkörper (2) und der Operationsstelle, und
ein Mittel (25) zum Auslösen der Änderung der individuellen Ansteuerung der Lichtstärken der Lichtquellen (5, 6), wobei
bei einer nachträglichen Veränderung des vorher gewählten Abstands zwischen dem Leuchtenkörper (2) und der Operationsstelle die Steuerungsvorrichtung (9) dazu angepasst ist, durch das Mittel (25) zum Auslösen ausgelöst die Lichtstärken der einzelnen Lichtquellen (5, 6) individuell so anzusteuern, dass der Durchmesser (dₓ) auf der Operationsstelle, bei dem die voreingestellte relative Beleuchtungsstärke (E_{cx}) des resultierenden Leuchtfelds (18) vorliegt, im Wesentlichen unverändert bleibt,
wobei für ein Verhindern einer Vergrößerung des Durchmessers (dₓ) die erste Lichtquelle (5), die das erste Leuchtfeld (14) mit dem kleineren Durchmesser (d1) erzeugt, so angesteuert wird, dass die Lichtstärke der ersten Lichtquelle (5) erhöht wird und/oder die zweite Lichtquelle (6), die das zweite Leuchtfeld (17) mit dem größeren Durchmesser (d2) erzeugt, so angesteuert wird, dass die Lichtstärke der zweiten Lichtquelle (6) verringert wird, und
wobei für ein Verhindern einer Verkleinerung des Durchmessers (dₓ) die erste Lichtquelle (5), die das erste Leuchtfeld (14) mit dem kleineren Durchmesser (d1) erzeugt, so angesteuert wird, dass die Lichtstärke der ersten Lichtquelle (5) verringert wird und/oder die zweite Lichtquelle (6), die das zweite Leuchtfeld (17) mit dem größeren Durchmesser (d2) erzeugt, so angesteuert wird, dass die Lichtstärke der zweiten Lichtquelle (6) erhöht wird.

2. Operationsleuchte (1) gemäß Anspruch 1, **dadurch gekennzeichnet, dass** die Steuerungsvorrichtung (9) dazu angepasst ist, dass bei einer Veränderung des Abstands eine normgerechte zentrale Beleuchtungsstärke (E_{c}) des resultierenden Leuchtfelds (18) unverändert bleibt.

3. Operationsleuchte (1) gemäß Anspruch 1 oder 2, **dadurch gekennzeichnet, dass** zumindest ein Teil der Lichtquellen (5, 6) verkippbar in dem Leuchtenkörper (2) angeordnet sind.

4. Operationsleuchte (1) gemäß einem der Ansprüche 1 oder 2, **dadurch gekennzeichnet, dass** die Operationsleuchte (1) mehrere Module aufweist, in denen jeweils mindestens eine der ersten Lichtquellen (5) und mindestens eine der zweiten Lichtquellen (6) angeordnet sind, wobei die Module zueinander kippbar sind.

5. Operationsleuchte (1) gemäß Anspruch 1 oder 2, **dadurch gekennzeichnet, dass**
eine Lichtaustrittsfläche (29) des Leuchtenkörpers (2) in einen im Wesentlichen kreisförmigen inneren Bereich (I) und mindestens einen darum herum angeordneten äußeren Bereich (11) unterteilt ist,
wobei die ersten und zweiten Lichtquellen (5, 6) starr angeordnet zumindest in dem inneren Bereich (I) vorhanden sind, und
mindestens eine dritte Lichtquelle (7) und mindestens eine vierte Lichtquelle (8), die jeweils ein Leuchtfeld mit unterschiedlichen Durchmessern bilden, in dem mindestens einen äußeren Bereich (II) vorgesehen sind, und
die dritten und vierten Lichtquellen (7, 8) in dem Leuchtenkörper (2) verkippbar gelagert sind und eine über die Steuerungsvorrichtung (9) gesteuerte Antriebsvorrichtung (10) zum Kippen der dritten und vierten Lichtquellen (7, 8) um einen Kippwinkel aufweisen, wobei
die Steuerungsvorrichtung (9) dazu angepasst ist, die Lichtstärken der dritten Lichtquellen (7) und der vierten Lichtquellen (8) und die Antriebsvorrichtung (10) so anzusteuern, dass der Durchmesser (dₓ), bei dem die voreingestellte relative Beleuchtungsstärke (E_{cx}) des resultierenden Leuchtfelds (18) vorliegt, im Wesentlichen unverändert bleibt.

6. Operationsleuchte (1) gemäß einem der Ansprüche 1 bis 5, **dadurch gekennzeichnet, dass** das Mittel (25) zum Auslösen der Änderung der individuellen Ansteuerung der Lichtstärken ein Bewegungssensor ist, und die Steuerungsvorrichtung (9) so ausgebildet ist, dass sie nach einer von dem Bewegungssensor erfassten abgeschlossenen Bewegung des Leuchtenkörpers (2) den erfassten Abstand zwischen dem Leuchtenkörper (2) und der Operationsstelle auswertet und die Lichtquellen (5, 6, 7, 8) entsprechend ansteuert.

7. Operationsleuchte (1) gemäß einem der vorangehenden Ansprüche, **dadurch gekennzeichnet, dass** die erste Lichtquelle (5) eine erste Linse aufweist und die zweite Lichtquelle (6) eine zweite Linse aufweist, und die erste Linse und die zweite Linse jeweils unterschiedliche optisch wirksame Flächen aufweisen, die so angepasst sind, dass die erzeugten Lichtfelder unterschiedliche Lichtverteilungen aufweisen.

8. Operationsleuchte (1) gemäß einem der vorangehenden Ansprüche, **dadurch gekennzeichnet, dass** die erste Lichtquelle (5) eine erste Linse aufweist und die zweite Lichtquelle (6) eine zweite Linse aufweist, und die erste Linse und die zweite Linse jeweils unterschiedliche Durchmesser aufweisen.

9. Operationsleuchte (1) gemäß einem der vorangehenden Ansprüche, **dadurch gekennzeichnet, dass** die Operationsleuchte (1) mindestens ein mit der Steuerungsvorrichtung (9) verbundenes Eingabemittel (22) zum Einstellen des Durchmessers (dₓ), bei dem die voreingestellte relative Beleuchtungsstärke (E_{cx}) des resultierenden Leuchtfelds (18) vorliegt, aufweist.

10. Operationsleuchte (1) gemäß Anspruch 9, **dadurch gekennzeichnet, dass** das Eingabemittel (22) ein Mittel zum Auswählen aus verschiedenen auswählbar voreingestellten Durchmessern (dₓ), bei denen die voreingestellte relative Beleuchtungsstärke (E_{cx}) vorliegt, ist.

11. Operationsleuchte (1) gemäß einem der vorangehenden Ansprüche, **dadurch gekennzeichnet, dass** die Lichtquellen (5, 6, 7, 8) in Gruppen zusammengefasst sind, wobei zumindest ein Kriterium für die Gruppierung der Durchmesser (d1, d2) des durch die Lichtquellen (5, 6, 7, 8) erzeugten Leuchtfelds (14, 17) und ein weiteres Kriterium der Abstand der Lichtquelle (5, 6, 7, 8) von der optischen Achse (11) ist, und die Steuerungsvorrichtung (9) so angepasst ist, dass die Lichtquellen (5, 6, 7, 8) in den einzelnen Gruppen jeweils gleich und die Gruppen individuell angesteuert werden können.

12. Operationsleuchte (1) gemäß Anspruch 5 und einem der Ansprüche 6 bis 11, **dadurch gekennzeichnet, dass** die dritten und vierten Lichtquellen (7, 8) im äußeren Bereich (II) angeordnet sind.

13. Operationsleuchte (1) gemäß einem der Ansprüche 11 oder 12, **dadurch gekennzeichnet, dass** die Steuerungsvorrichtung (9) einen Speicherbereich aufweist, und die Steuerungsvorrichtung (9) so ausgebildet ist, dass die Lichtstärken der Lichtquellen (5, 6, 7, 8) in den einzelnen Gruppen als Bestromungsstärke in Form eines Kennfelds in dem Speicherbereich hinterlegt sind und abhängig von dem Abstand zwischen dem Leuchtenkörper (2) und der Operationsstelle als Mischungsverhältnis von der Steuerungsvorrichtung abrufbar sind.

14. Operationsleuchte (1) gemäß Anspruch 5 und Anspruch 13, **dadurch gekennzeichnet, dass** die Steuerungsvorrichtung (9) so ausgebildet ist, dass der Kippwinkel in dem Speicherbereich in Abhängigkeit von dem Abstand zwischen dem Leuchtenkörper (2) und der Operationsstelle gespeichert ist, und von der Steuerungsvorrichtung (9) entsprechend dem Abstand zwischen dem Leuchtenkörper (2) und der Operationsstelle abrufbar ist.

15. Verfahren zum Betreiben einer Operationsleuchte (1) gemäß einem der vorangehenden Ansprüche, mit den folgenden Schritten:
Erfassen einer Änderung eines Abstands zwischen dem Leuchtenkörper (2) und der Operationsstelle mit der vorrichtung (24) zum Erfassen des Abstands zwischen dem Leuchtenkörper (2) und der Operationsstelle,
Auslösen des Anpassens des Durchmessers (dₓ), bei dem die voreingestellte relative Beleuchtungsstärke (E_{cx}) des resultierenden Leuchtfelds (18) vorliegt, nach dem Beendigen der Änderung des Abstands, durch Ändern der jeweiligen Lichtstärke der mindestens ersten Lichtquelle (5) und der mindestens zweiten Lichtquelle (6), so dass der vor der Änderung des Abstandes eingestellte Durchmesser (dₓ), bei dem die voreingestellte relative Beleuchtungsstärke (E_{cx}) des resultierenden Leuchtfelds (18) vorliegt, im Wesentlichen unverändert bleibt.

16. Verfahren gemäß Anspruch 15,
wobei der gewünschte Durchmesser (dₓ), bei dem die voreingestellte relative Beleuchtungsstärke (E_{cx}) des resultierenden Leuchtfelds (18) vorliegt, vorab eingestellt wird.

17. Verfahren gemäß einem der Ansprüche 15 oder 16,
wobei die gewünschte zentrale Beleuchtungsstärke (E_{c}) des resultierenden Leuchtfelds (18) vorab eingestellt wird.

18. Verfahren gemäß einem der Ansprüche 15 bis 17,
wobei für ein Verhindern einer Vergrößerung des Durchmessers (dₓ), bei dem die voreingestellte relative Beleuchtungsstärke (E_{cx}) des resultierenden Leuchtfelds (18) vorliegt, die erste Lichtquelle (5), die das erste Leuchtfeld (14) mit dem kleineren Durchmesser (d1) erzeugt, so angesteuert wird, dass die Lichtstärke der ersten Lichtquelle (5) erhöht wird und/oder die zweite Lichtquelle (6), die das zweite Leuchtfeld (17) mit dem größeren Durchmesser (d2) erzeugt, so angesteuert wird, dass die Lichtstärke der zweiten Lichtquelle (6) verringert wird, und
wobei für ein Verhindern einer Verkleinerung des Durchmessers (dₓ), bei dem die voreingestellte relative Beleuchtungsstärke (E_{cx}) des resultierenden Leuchtfelds (18) vorliegt, die erste Lichtquelle (5), die das erste Leuchtfeld (14) mit dem kleineren Durchmesser (d1) erzeugt, so angesteuert wird, dass die Lichtstärke der ersten Lichtquelle (5) verringert wird und/oder die zweite Lichtquelle (6), die das zweite Leuchtfeld (17) mit dem größeren Durchmesser (d2) erzeugt, so angesteuert wird, dass die Lichtstärke der zweiten Lichtquelle (6) erhöht wird.

## Claims

1. Surgical lamp (1) for illuminating a surgery site arranged in a chosen distance comprising:
a lamp body (2) having an optical axis (11), comprising at least one first light source (5) and one second light source (6), wherein the first light source (5) emits a light sheaf (12) having a first axis (13) intersecting the optical axis (11) in a distance (1₁) from the lamp body (2), the second light source (6) emits a light sheaf (15) having a second axis (16) intersecting in the same distance (1₁) from the lamp body (2) with its optical axis (11), the first light source (5) generates a first light field (14), and the second light source (6) generates a second light field (17) with respective different diameters (d₁, d₂) on the surgery site, and the light fields (14, 17) result in a resulting light field (18) which is basically circular and which has a light distribution conforming to the surgical lamp standard, at the moment DIN EN 60601-2-41:2010, with a preset relative illuminance (E_{cx}) conforming to the standard on a predetermined diameter (dₓ),
a control device (9) for the light sources (5, 6),
driving units (28) via which the light sources (5, 6) are respectively connected to the control device (9), wherein the control device (9) is configured to individually control the light intensity of the first light source (5) and the light intensity of the second light source (6) so that the preset relative illuminance (E_{cx}) exists on the predetermined diameter (dₓ) in the chosen distance,
a device (24) for detecting the distance between the lamp body (2) and the surgery site, and
a means (25) for triggering the change of the individual control of the light intensities of the light sources (5, 6),
wherein
the control device (9) is configured, triggered by the means (25) for triggering, to individually control the light intensities of the individual light sources (5, 6) such that the diameter (dₓ) on the surgery site where the preset relative illuminance (E_{cx}) of the resulting light field (18) exists is kept substantially unchanged when the previously chosen distance between the lamp body (2) and the surgery site is subsequently changed,
wherein, for preventing an enlargement of the diameter (dₓ), the first light source (5) generating the first light field (14) having the smaller diameter (d1) is controlled such that the light intensity of the first light source (5) is enlarged, and/or the second light source (6) generating the second light field (17) having the larger diameter (d2) is controlled such that the light intensity of the second light source (6) is reduced, and
wherein for preventing a reduction of the diameter (dₓ), the first light source (5) generating the first light field (14) having the smaller diameter (d1) is controlled such that the light intensity of the first light source (5) is reduced, and/or the second light source (6) generating the second light field (17) having the larger diameter (d2) is controlled such that the light intensity of the second light source (6) is enlarged.

2. Surgical lamp (1) according to claim 1, **characterized in that** the control device (9) is configured to keep a central illuminance (Eₑ) conforming to the standard of the resulting light field (18) unchanged when the distance is changed.

3. Surgical lamp (1) according to claim 1 or 2, **characterized in that** at least a part of the light sources (5, 6) is tiltably arranged in the lamp body (2).

4. Surgical lamp (1) according to any of the claims 1 or 2, **characterized in that** the surgical lamp (1) comprises several modules in which at least one of the first light sources (5) and at least one of the second light sources (6) are respectively arranged, wherein the modules are tiltable with respect to each other.

5. Surgical lamp (1) according to claim 1 or 2, **characterized in that**
a light emerging surface (29) of the lamp body (2) is divided in a an inner area (I) which is basically circular and in at least one outer area (II) arranged around the inner area (I),
wherein the first and second light sources (5, 6) exist rigidly arranged at least in the inner area (I), and
at least one third light source (7) and at least one fourth light source (8) respectively generating a light field having different diameters are provided in the at least one outer area (II), and
the third and fourth light sources (7, 8) are tiltably supported in the lamp body (2) and comprise a driving device (10), controlled by the control device (9), for tilting the third and fourth light sources (7, 8) about a tilting angle, wherein
the control device (9) is configured to control the light intensities of the third light sources (7) and of the fourth light sources (8) and the driving device (10) such that the diameter (dₓ) where the preset relative illuminance (E_{cx}) of the resulting light field (18) exists is kept substantially unchanged.

6. Surgical lamp (1) according to any of the claims 1 to 5, **characterized in that** the means (25) for triggering the change of the individual control of the light intensities is a motion sensor, and the control device (9) is configured such that it evaluates the detected distance between the lamp body (2) and the surgery site and that it appropriately controls the light sources (5, 6, 7, 8) after a completed motion of the lamp body (2) detected by the motion sensor.

7. Surgical lamp (1) according to any of the preceding claims, **characterized in that** the first light source (5) comprises a first lens and the second light source (6) comprises a second lens, and the first lens and the second lens respectively comprise different optically operative surfaces being configured such that the generated light fields have different light distributions.

8. Surgical lamp (1) according to any of the preceding claims, **characterized in that** the first light source (5) comprises a first lens and the second light source (6) comprises a second lens, and that the first lens and the second lens respectively comprise different diameters.

9. Surgical lamp (1) according to any of the preceding claims, **characterized in that** the surgical lamp (1) comprises at least one input means (22) connected to the control device (9) for setting the diameter (dₓ) where the preset relative illuminance (E_{cx}) of the resulting light field (18) exists.

10. Surgical lamp (1) according to claim 9, **characterized in that** the input means (22) is a means for selecting among different selectably preset diameters (dₓ) where the preset relative illuminance (E_{cx}) exists.

11. Surgical lamp (1) according to any of the preceding claims, **characterized in that** the light sources (5, 6, 7, 8) are grouped in groups, wherein at least one criterion for the grouping is the diameter (d1, d2) of the light field (14, 17) generated by the light sources (5, 6, 7, 8) and a further criterion is the distance of the light source (5, 6, 7, 8) from the optical axis (11), and the control device (9) is configured such that the light sources (5, 6, 7, 8) can be controlled respectively similar in the individual groups and the groups can be controlled individually.

12. Surgical lamp (1) according to claim 5 and any of the claims 6 to 11, **characterized in that** the third and fourth light sources (7, 8) are arranged in the outer area (II).

13. Surgical lamp (1) according to any of the claims 11 or 12, **characterized in that** the control device (9) comprises a memory section, and the control device (9) is configured such that the light intensities of the light sources (5, 6, 7, 8) in the individual groups are stored as force of current in the form of a mapping in the memory section and that they are retrievable by the control device as mixing ratio depending on the distance between the lamp body (2) and the surgery site.

14. Surgical lamp (1) according to claim 5 and claim 13, **characterized in that** the control device (9) is configured such that the tilting angle is stored in the memory section depending on the distance between the lamp body (2) and the surgery site, and that it is retrievable by the control device (9) according to the distance between the lamp body (2) and the surgery site.

15. Method for surgery a surgical lamp (1) according to any of the preceding claims, with the following steps:
detecting a change of a distance between the lamp body (2) and the surgery site by the device (24) for detecting the distance between the lamp body (2) and the surgery site,
triggering the adjustment of the diameter (dₓ) where the preset relative illuminance (E_{cx}) of the resulting light field (18) exists after the completion of the change of the distance by altering the respective light intensity of the at least first light source (5) and the at least second light source (6) so that the diameter (dₓ) where the preset relative illuminance (E_{cx}) previously set to the change of the distance exists is kept substantially unchanged.

16. Method according to claim 15,
wherein the requested diameter (dₓ) at which the preset relative illuminance (E_{cx}) of the resulting light field (18) exists is set in advance.

17. Method according to any of claims 15 or 16,
wherein the requested central illuminance (E_{c}) of the resulting light field (18) is set in advance.

18. Method according to any of the claims 15 to 17,
wherein, for preventing an enlargement of the diameter (dₓ) at which the preset relative illuminance (E_{cx}) of the resulting light field (18) exists, the first light source (5) generating the first light field (14) having the smaller diameter (d1) is controlled such that the light intensity of the first light source (5) is enlarged, and/or the second light source (6) generating the second light field (17) having the larger diameter (d2) is controlled such that the light intensity of the second light source (6) is reduced, and
wherein for preventing a reduction of the diameter (dₓ) at which the preset relative illuminance (E_{cx}) of the resulting light field (18) exists, the first light source (5) generating the first light field (14) having the smaller diameter (d1) is controlled such that the light intensity of the first light source (5) is reduced, and/or the second light source (6) generating the second light field (17) having the larger diameter (d2) is controlled such that the light intensity of the second light source (6) is enlarged.

## Revendications

1. Lampe d'opération (1) permettant d'éclairer une zone opératoire située à une distance choisie, comportant :
un corps de lampe (2) avec un axe optique (11), qui comporte au moins une première source lumineuse (5) et une deuxième source lumineuse (6), ladite première source lumineuse (5) émettant un faisceau de lumière (12), qui possède un premier axe (13) qui coupe l'axe optique (11) à une distance (1₁) du corps de lampe (2), ladite deuxième source lumineuse (6) émettant un faisceau de lumière (15) qui possède un deuxième axe (16) qui coupe l'axe optique (11) du corps de lampe (2) à la même distance (1₁) de celui-ci, ladite source lumineuse (5) formant un premier champ lumineux (14) et la deuxième source lumineuse (6) formant sur la zone opératoire un deuxième champ lumineux (17) avec chacun un diamètre (d1, d2) différent, et les champs lumineux (14, 17) donnant un champ lumineux (18) résultant sensiblement circulaire avec une répartition de la lumière conforme à la norme relative aux lampes d'opération, actuellement DIN EN 60601-2-41 : 2010, avec une intensité d'éclairage (E_{cx}) relative préréglée, conforme à la norme, en présence d'un diamètre (dₓ) prédéterminé,
un dispositif de commande (9) pour les sources lumineuses (5, 6),
des unités d'activation (28), par l'intermédiaire desquelles les sources lumineuses (5, 6) sont reliées respectivement au dispositif de commande (9), ledit dispositif de commande (9) étant ajusté pour commander individuellement l'intensité lumineuse de la première source lumineuse (5) et l'intensité lumineuse de la deuxième source lumineuse (6), de telle sorte qu'avec la distance choisie l'intensité d'éclairage (E_{cx}) relative préréglée existe en présence du diamètre (dₓ) prédéterminé,
un dispositif (24) destiné à détecter la distance entre le corps de lampe (2) et la zone opératoire, et
un moyen (25) destiné à déclencher la modification de la commande individuelle des intensités lumineuses des sources lumineuses (5, 6),
en présence d'une modification ultérieure de la distance réglée au préalable entre le corps de lampe (2) et la zone opératoire, le dispositif de commande (9) étant ajusté, en étant déclenché via le moyen (25) destiné à déclencher, pour commander individuellement les intensités lumineuses des différentes sources lumineuses (5, 6), de telle sorte que le diamètre (dₓ) sur la zone opératoire, en présence duquel existe l'intensité d'éclairage (E_{cx}) relative préréglée du champ lumineux (18) résultant, reste sensiblement inchangé,
pour empêcher un agrandissement du diamètre (dₓ), la première source lumineuse (5), qui génère le premier champ lumineux (14) avec le diamètre (d1) plus petit, étant commandée de telle sorte que l'intensité lumineuse de la première source lumineuse (5) est accrue, et/ou la deuxième source lumineuse (6), qui génère le deuxième champ lumineux (17) avec le diamètre (d2) plus grand, étant commandée de telle sorte que l'intensité lumineuse de la deuxième source lumineuse (6) est diminué, et
pour empêcher une diminution du diamètre (dₓ), la première source lumineuse (5), qui génère le premier champ lumineux (14) avec le diamètre (d1) plus petit, étant commandée de telle sorte que l'intensité lumineuse de la première source lumineuse (5) est diminuée, et/ou la deuxième source lumineuse (6), qui génère le deuxième champ lumineux (17) avec le diamètre (d2) plus grand, étant commandée de telle sorte que l'intensité lumineuse de la deuxième source lumineuse (6) est accrue.

2. Lampe d'opération (1) selon la revendication 1, **caractérisée en ce que** le dispositif de commande (9) est ajusté pour que, en cas de modification de la distance, une intensité d'éclairage (E_{c}) centrale, conforme à la norme, du champ lumineux (18) résultant reste inchangée.

3. Lampe d'opération (1) selon la revendication 1 ou 2, **caractérisée en ce qu'**au moins une partie des sources lumineuses (5, 6) sont disposées dans le corps de lampe (2) de manière à pouvoir basculer.

4. Lampe d'opération (1) selon la revendication 1 ou 2, **caractérisée en ce que** la lampe d'opération (1) comporte plusieurs modules, dans chacun desquels sont disposées au moins une des premières sources lumineuses (5) et au moins une des deuxièmes sources lumineuses (6), les modules étant aptes à basculer les uns par rapport aux autres.

5. Lampe d'opération (1) selon la revendication 1 ou 2, **caractérisée en ce que**
une surface de sortie de lumière (29) du corps de lampe (2) est divisée en une zone intérieure (I) sensiblement circulaire et en au moins une zone extérieure (II) disposée autour de cette dernière,
les premières et les deuxièmes sources lumineuses (5, 6) étant présentes au moins dans la zone intérieure (I) en étant disposées de manière fixe, et
et il est prévu dans ladite au moins une zone extérieure (II) au moins une troisième source lumineuse (7) et au moins une quatrième source lumineuse (8) qui forment chacune un champ lumineux avec des diamètres différents, et
les troisième et quatrième sources lumineuses (7, 8) étant logées dans le corps de lampe (2) de manière à pouvoir basculer et comportant un dispositif d'entraînement (10), commandé par le dispositif de commande (9) et destiné à basculer les troisièmes et quatrièmes sources lumineuses (7, 8) selon un angle de basculement,
le dispositif de commande (9) étant ajusté pour commander les intensités lumineuses des troisièmes sources lumineuses (7) et des quatrièmes sources lumineuses (8) et le dispositif d'entraînement (10), de telle sorte que le diamètre (dₓ), en présence duquel existe l'intensité d'éclairage (E_{cx}) relative préréglée du champ lumineux (18) résultant, reste sensiblement inchangé.

6. Lampe d'opération (1) selon l'une quelconque des revendications 1 à 5, **caractérisée en ce que** le moyen (25) destiné à déclencher la modification de la commande individuelle des intensités lumineuses est un capteur de mouvement, et le dispositif de commande (9) est configuré de telle sorte que, à la suite d'un mouvement du corps de lampe (2) détecté par le capteur de mouvement, il analyse la distance détectée entre le corps de lampe (2) et la zone opératoire et commande les sources lumineuses (5, 6, 7, 8) de manière appropriée.

7. Lampe d'opération (1) selon l'une quelconque des revendications précédentes, **caractérisée en ce que** la première source lumineuse (5) comporte une première lentille et la deuxième source lumineuse (6) comporte une deuxième lentille, et la première lentille et la deuxième lentille comportent chacune des surfaces optiquement actives différentes, qui sont ajustées de telle sorte que les champs lumineux générés présentent des répartitions de lumière différentes.

8. Lampe d'opération (1) selon l'une quelconque des revendications précédentes, **caractérisée en ce que** la première source lumineuse (5) comporte une première lentille et la deuxième source lumineuse (6) comporte une deuxième lentille, et la première lentille et la deuxième lentille comportent chacune des diamètres différents.

9. Lampe d'opération (1) selon l'une quelconque des revendications précédentes, **caractérisée en ce que** la lampe d'opération (1) comporte au moins un moyen d'entrée (22), relié au dispositif de commande (9), pour régler le diamètre (dₓ), en présence duquel existe l'intensité d'éclairage (E_{cx}) relative préréglée du champ lumineux (18) résultant.

10. Lampe d'opération (1) selon la revendication 9, **caractérisée en ce que** le moyen d'entrée (22) est un moyen pour choisir parmi différents diamètres (dₓ) préréglés sélectionnables, pour lesquels existe l'intensité d'éclairage (E_{cx}) relative préréglée.

11. Lampe d'opération (1) selon l'une quelconque des revendications précédentes, **caractérisée en ce que** les sources lumineuses (5, 6, 7, 8) sont réunies en groupes, au moins un critère étant pour le regroupement des diamètres (d1, d2) du champ lumineux (14, 17) généré par les sources lumineuses (5, 6, 7, 8) et un autre critère étant la distance entre les sources lumineuses (5, 6, 7, 8) et l'axe optique (11), et le dispositif de commande (9) étant ajusté de telle sorte que les sources lumineuses (5, 6, 7, 8) dans les différents groupes peuvent être commandées de manière identique et les groupes peuvent être commandés individuellement.

12. Lampe d'opération (1) selon la revendication 5 et l'une quelconque des revendications 6 à 11, **caractérisée en ce que** les troisièmes et quatrièmes sources lumineuses (7, 8) sont disposées dans la zone extérieure (II).

13. Lampe d'opération (1) selon la revendication 11 ou 12, **caractérisée en ce que** le dispositif de commande (9) comporte une zone de mémoire, et le dispositif de commande (9) est configuré de telle sorte que les intensités lumineuses des sources lumineuses (5, 6, 7, 8) dans les différents groupes sont stockées sous la forme d'un diagramme caractéristique dans ladite zone de mémoire en tant qu'intensités de courant et, en fonction de la distance entre le corps de lampe (2) et la zone opératoire, peuvent être extraites de ladite mémoire par le dispositif de commande sous forme de rapport de mélange.

14. Lampe d'opération (1) selon les revendications 5 et la revendication 13, **caractérisée en ce que** le dispositif de commande (9) est configuré de telle sorte que l'angle de basculement est mémorisé dans la zone de mémoire en fonction de la distance entre le corps de lampe (2) et la zone opératoire et est extrait de ladite mémoire par la dispositif de commande (9) en fonction de la distance entre le corps de lampe (2) et la zone opératoire.

15. Procédé permettant d'utiliser une lampe d'opération (1) selon l'une quelconque des revendications précédentes, comportant les étapes suivantes :
détection d'une modification d'une distance entre le corps de lampe (2) et la zone opératoire au moyen du dispositif (24) destiné à détecter la distance entre le corps de lampe (2) et la zone opératoire,
déclenchement de l'ajustement du diamètre (dₓ), en présence duquel existe l'intensité d'éclairage (E_{cx}) relative préréglée du champ lumineux (18) résultant, à la fin de la modification de la distance, par la modification des intensités lumineuses respectives de ladite au moins une première source lumineuse (5) et de ladite au moins une deuxième source lumineuse (6), de telle sorte que le diamètre (dₓ), réglé avant la modification de la distance et en présence duquel existe l'intensité d'éclairage (E_{cx}) relative préréglée du champ lumineux (18) résultant, reste sensiblement inchangé.

16. Procédé selon la revendication 15, dans lequel le diamètre (dₓ) souhaité, en présence duquel existe l'intensité d'éclairage (E_{cx}) relative préréglée du champ lumineux (18) résultant, est réglé préalablement.

17. Procédé selon la revendication 15 ou 16, dans lequel l'intensité d'éclairage (E_{c}) centrale souhaitée du champ lumineux (18) résultant est réglée préalablement.

18. Procédé selon l'une quelconque des revendications 15 à 17,
dans lequel pour empêcher un agrandissement du diamètre (dₓ), en présence duquel existe l'intensité d'éclairage (E_{cx}) relative préréglée du champ lumineux (18) résultant, la première source lumineuse (5), qui génère le premier champ lumineux (14) avec le diamètre (d1) plus petit, est commandée de telle sorte que l'intensité lumineuse de la première source lumineuse (5) est accrue, et/ou la deuxième source lumineuse (6), qui génère le deuxième champ lumineux (17) avec le diamètre (d2) plus grand, est commandée de telle sorte que l'intensité lumineuse de la deuxième source lumineuse (6) est diminué, et
dans lequel pour empêcher une diminution du diamètre (dₓ), en présence duquel existe l'intensité d'éclairage (Eₑₓ) relative préréglée du champ lumineux (18) résultant, la première source lumineuse (5), qui génère le premier champ lumineux (14) avec le diamètre (d1) plus petit, est commandée de telle sorte que l'intensité lumineuse de la première source lumineuse (5) est diminuée, et/ou la deuxième source lumineuse (6), qui génère le deuxième champ lumineux (17) avec le diamètre (d2) plus grand, est commandée de telle sorte que l'intensité lumineuse de la deuxième source lumineuse (6) est accrue.
